# EUROPEAN PATENT APPLICATION

(11) **EP 2 317 451 A2**
(43) Date of publication of application: **04.05.2011**
(21) Application number: 10186467.6
(22) Date of filing: 04.10.2010
(51) Int. Cl.: G06F 19/00, A61M 5/172

(54) **Method and system for delivering analgesic drugs**

(30) Priority: 06.10.2009 US 574327
(71) Applicant: General Electric Company, Schenectady, NY 12345 (US)
(72) Inventor: Uutela, Kimmo Henrik, 00100, Helsinki (FI); Viertio-Oja, Hanna Elina, 02660, Espoo (FI); Virtanen, Juha Petri, 00660, Helsinki (FI)
(74) Representative: Illingworth-Law, William Illingworth

(57) **Abstract**

An analgesic drug delivery system is disclosed herein. The system includes a drug demand interface (110), a patient parameter monitoring device (120), a control system (130), and a drug delivery system (140). The drug demand interface (110) obtains a drug demand from a patient. The patient parameter monitoring device (120) measures pain level of the patient. The control system (130) receives input from the patient parameter monitoring device (120) and the drug demand interface (110) and controls the drug delivery based on effectiveness of the drug. The drug delivery system (140) administers the drug in a controlled manner under the control of the control system (130).

## Description

### FIELD OF INVENTION

This invention relates generally to patient care systems and methods, and more particularly, to a system and a method for controlling the self-administration of analgesic drugs to a patient based on effectiveness of a drug.

### BACKGROUND OF INVENTION

Patient controlled analgesia (PCA) devices are common and are used to deliver patent analgesic drug based on some predetermined rules. The patient sends his requirements and based on the demand, bolus doses of analgesic drug is delivered to the patient.

Different parameters are measured while infusing the drug through a PCA. Mostly, physiological parameters of the patient are being measured to ensure the patient safety. Some of the examples include monitoring SpO2 or ETCO2, while infusing the drug. The analgesic, as well as sedative and anesthetic drugs, may affect the central nervous system or respiratory system. Thus during drug infusion, parameters such as SpO2 or ETCO2 may be measured to ensure the safety of central nervous system or respiratory system.

However, it will be beneficial to measure the efficiency or effectiveness of a drug in the patient. Conventionally, after a drug is administered to the patient, patient parameters are monitored to ensure the patient safety. When the patient feels uncomfortable, he demands for more drugs and based on some preset rules, the drug is delivered to the patient. This process has the disadvantage that the patient feels the pain and then a drug demand request is placed. Once the system receives the drug demand request, the drug dosage is calculated and delivered to the patient. By that time the system delivers the drug to the patient and the drug starts affecting the patient, the patient must have suffered considerable pain.

Further, different drugs may have slightly varying effect on different patients and it will be beneficial to measure the effectiveness of the drug before administering more drugs to the patient.

Thus there exist a need to provide an improved method for delivering analgesic drug to a patient before the patient feels too uncomfortable.

### SUMMARY OF INVENTION

The above-mentioned shortcomings, disadvantages and problems are addressed herein which will be understood by reading and understanding the following specification.

One embodiment of the present invention provides a method of delivering an analgesic drug. The method comprises: administering analgesic drug to a patient based on input from a patient controlled input device; continuously measuring pain level of the patient with reference to at least one patient parameter; receiving drug demand from the patient through the patient controlled input device; and controlling administration of the analgesic drug based on the measured pain level and the received drug demand.

In another embodiment, an analgesic drug delivery system is disclosed. The system comprises: a drug demand interface obtaining drug demand request from a patient; a patient parameter monitoring device measuring pain level of the patient; a control system configured to receive input from patient parameter monitoring device and the drug demand interface and configured to control the drug delivery based on the effectiveness of the drug; and a drug delivery system configured to administer the drug in a controlled manner under the control of the control system.

In another embodiment, an improved patient controlled analgesia (PCA) device is disclosed. The device comprises: patient controlled input device associated with a patient configured to communicate patient request about drug demand; patient monitoring device for measuring pain level of a patient continuously; processor for checking effectiveness of the drug and processing the patient request with reference to the measured pain level and effectiveness of the drug; drug delivery device associated with the processor configured to administer drug under the control of the processor such that the drug is administered before the patient is uncomfortable.

Various other features, objects, and advantages of the invention will be made apparent to those skilled in the art from the accompanying drawings and detailed description thereof.

### BRIEF DESCRIPTION OF THE DRAWINGS

**FIG. 1** is a block diagram of a drug delivery system as described in an embodiment of the invention;

**FIG. 2** is a diagrammatic representation of a drug delivery system that controls the drug delivery with reference to the patient's pain level and drug demand as described in an embodiment of the invention;

**FIG. 3** is a block diagram of an improved patient controlled analgesia system as described in an embodiment of the invention;

**FIG. 4** is a flowchart illustrating an analgesic drug delivery method as described in an embodiment of the invention; and

**FIG. 5** is a detailed flowchart illustrating an analgesic drug delivery method as described in an embodiment of the invention.

### DETAILED DESCRIPTION OF INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention. To the extent that the figures illustrate diagrams of the functional blocks of various embodiments, the functional blocks are not necessarily indicative of the division between hardware circuitry. Thus, for example, one or more of the functional blocks (e.g., processors or memories) may be implemented in a single piece of hardware (e.g., a general purpose signal processor or a block of random access memory, hard disk, or the like). Similarly, the programs may be stand alone programs, may be incorporated as subroutines in an operating system, may be functions in an installed software package, and the like. It should be understood that the various embodiments are not limited to the arrangements and instrumentality shown in the drawings.

Embodiments of the present invention provide a method and system for controlling delivery of an analgesic drug based on the effectiveness of a drug on patient and/or based on patient's pain level. The invention improves the PCA device by configuring it to deliver drug based on the effectiveness of the drug.

The term "patient parameter" referred in the specification needs not be limited to the patient pain level. It could include different other parameters that could express the pain level or any other parameter that could indicate the pain level or effectiveness of the drug. Further the "pain level" could be determined by different parameters and need not be limited to the examples mentioned in the specification. Similarly the term "analgesic" drug could include any analgesic, sedative, anesthetic drug.

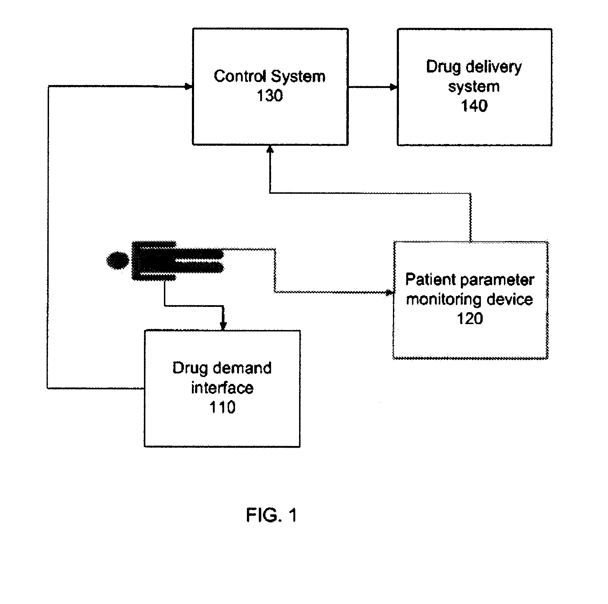

**FIG. 1** is a block diagram of a drug delivery system as described in an embodiment of the invention. The drug delivery system comprises a drug demand interface 110, patient parameter monitoring device 120, a control system 130 and a drug delivery system 140. The drug demand interface 110 is provided with a patient who needs to be given analgesic, sedative or anesthetic drugs. The drug demand interface 110 could be operated by the patient himself or by a caretaker based on the patient's instructions. The drug demand interface 110 could be provided to the patient and he could operate the same whenever he feels pain. The drug demand interface 110 communicates the patient drug demand to the control system 130 either through a cable or wirelessly. In an embodiment, the drug interface may be a part of a PCA (Patient controlled analgesia) device.

The patient monitoring parameter device 120 is configured to monitor physiological parameters of a patient. The physiological parameters could include Surgical Stress Index (SSI), heart rate, skin conductivity, blood pressure, pupil size and forehead muscle activity. However any physiological parameter or gestures of the patient that communicates the pain level or the effectiveness of the drug may be monitored. These parameters could be measured using various patient monitoring devices. Monitoring gestures for identifying pain level could include monitoring at least one of facial expressions, sound, movements of body parts, activity, cry, and consolability of the patient. The physiological parameters may be measured continuously after administering the drug or may be at a predetermined interval based on the requirement. Different physiological parameters may be measured to determine the pain level of the patient. The measured patient parameter should help in calculating the effectiveness of the drug. The effectiveness of the drug may be determined by patient's pain level, sleeping pattern, facial expressions, other gestures etc. However, the examples need not be limited these.

The measured patient parameter and the input from drug demand interface 110 are given to the control system 130. Based on the inputs, the control system 130 decides on the drug delivery and calculates the drug dosage, if required. The control system 130 may use certain rules while calculating the drug dosage. The rules could include previous drug demand, properties or side effects of drug, maximum threshold of drug level in blood, maximum or minimum pain level threshold for the patient etc. The control unit analyzes the drug level of the patient using different measured parameters and pre-set rules.

In an embodiment, the control unit 130 is configured to set rules based on the monitored parameters. The control unit adjusts the patient's pain threshold level based on the monitored pain level or the drug demand. This could be done by monitoring the amount of drug in the patient's blood level and setting the threshold value based on that.

In an embodiment, the patient parameter monitoring device 120 identifies the amount of drug present in the patient's blood. If the drug level in the patient's blood is low, the drug delivery may be controlled based on that information. In an example, the drug may be delivered automatically, if the drug level in patient's blood is below a threshold level. In another example, the system may wait for the drug demand from the patient and upon receiving the same, the drug may be delivered. In this event, the system need not have to wait for the patient drug demand to access the pain level or drug level in the blood for calculating the dosage. The dosage may be calculated based on the pain level or drug level in advance and administered to the patient immediately upon receiving the request from the patient.

Similarly, if the pain level of the patient goes below a minimum threshold level, the system can abort the drug administration. The control system 130 can also learn the minimum value of SSI (Surgical Stress Index), or other hemodynamic markers, that lead to drug demands, and thus decrease drug infusion if SSI drops below a personalized threshold. The threshold levels could also be dependent on the circadian rhythm or on the trends of SSI and drug concentration and by monitoring these parameters the drug administration can be controlled.

The control system 130 is configured to provide a drug delivery control signal to the drug delivery system 140. The drug delivery system 140 helps in administering the drug to the patient. The drug delivery system 140 could include infusion pumps. The drug delivery system 140 could automatically deliver the drug to the patient upon receiving signal from the control system 130. Alternately, the control system 130 may inform a caretaker and caretaker may control the drug delivery system 140.

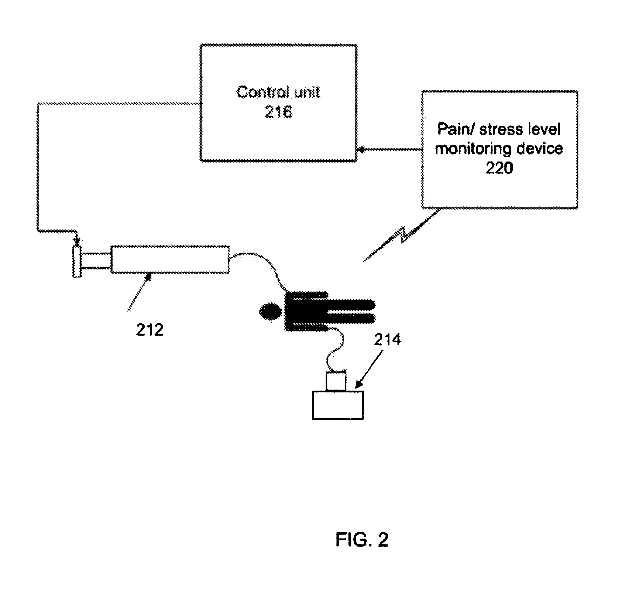

**FIG. 2** is a diagrammatic representation of a drug delivery system that controls the drug delivery with reference to the patient's pain level and drug demand as described in an embodiment of the invention. In an embodiment, a PCA (Patient controlled analgesia) device is used to deliver analgesic drug to a patient based on his demand. The PCA device includes a drug infusion pump 212 and a drug demand interface 214 configured to communicate the drug demands of the patient. In an embodiment, the drug demand interface 214 is a communication device provided with an interface such as a button and by pressing the button the patient communicates the drug requirement. The PCA device further comprises a control unit 216 configured to control the drug delivery based on the patient drug demand.

The system further comprises a pain/stress level monitoring device 220 configured to monitor the patient pain level after a drug is administrated to the patient. The pain level monitoring device may identify the pain level by monitoring certain physiological parameter and/ or by monitoring the gestures of the patient. The monitored physiological parameter or the patient gestures is converted to a numerical value corresponding to the patient pain level. The monitored pain level is provided to the control unit 216. Alternately the pain level monitoring device may communicate the pain level to the patient and the control unit 216 may derive corresponding numerical value. The control unit 216 calculates the drug dosage based on the drug demand from the patient and the monitored patient pain level.

In an embodiment, a pharmacokinetic and pharmacodynamic (PK/PD) model is used to estimate the blood concentration of the drug and its effect based on the history of drug administrations and general patient information, such as weight, height, and gender. Using these models the control unit 256 could identify the amount of drug present in patient's blood. These models can be used, for example, to calculate an appropriate dose needed to keep the pain measurements at low levels. The control unit 216 may calculate the drug dosage based on at least one of the current drug level in the patient, pain level of the patient and the patient drug demand.

In an embodiment, based the measured pain level and the amount of the drug in the blood, the effectiveness of the drug can be calculated. Further effectiveness of the drug may be calculated based on the previous drug demands such as the frequency at which the patient is demanding the drug, dosage given at each time etc. The effectiveness of the drug is calculated by the control unit 216 and based on the same, the control unit 216 calculates the drug dosage.

Based on the effectiveness of the drug, the dosage or the frequency of administration could be adjusted. This will help the patient to receive drug before he realizes the pain. Thus the system calculates the effectives of the drug and based on the same and the patient drug demand, drug administration is controlled.

Based on the calculated dosage, the drug infusion pump 212 infuses the drug to the patient.

In an embodiment, the control unit 216 need not be associated with the PCA device. The control unit 216 may be any processor capable of receiving inputs from drug demand interface and the pain level monitoring device 220 and controlling the drug administration.

In an embodiment, based on the measured pain level or the effectiveness of the drug, the threshold value of the patient may be adjusted. For, example, it could be determined that how effectively patient is responding to the drugs and based on that the minimum/ maximum pain level threshold could be adjusted.

Upon noticing the pain level above the maximum personal threshold value, the system may automatically infuse drug without waiting for the patient to send the drug demand request. This will be helpful if the patient is sleeping or in a condition where he is not able to operate the drug demand interface. Else the system may calculate the drug dosage in advance and upon receiving the drug demand request quickly, infuse the drug to the patient.

Upon noticing the drug level in the blood above the maximum allowable threshold level, or the pain level below the minimum threshold, the system may abort the drug infusion.

If a drug is not found effective for a patient, for example, even after administering the drug if the patient pain level is not coming down, the system may inform the clinician or the caretaker and the caretaker may try an alternate drug or adjust the dosage.

Instead of patient pain level, different other parameters such as SSI could be monitored and measured. Based on the monitored SSI, the drug administration could be controlled.

Further the effectiveness of the drug studied on the patient may be recorded for future use.

Thus various embodiments of the invention help to administer drug to patient before the patient is too uncomfortable.

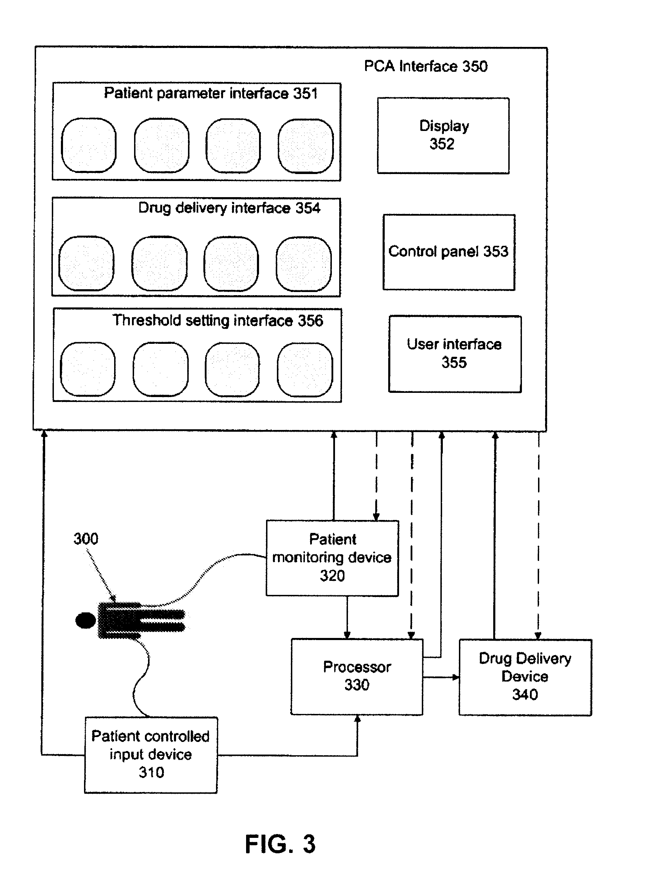

**FIG. 3** is a block diagram of an improved patient controlled analgesia device as described in an embodiment of the invention. The PCA device is provided with a patient controlled input device 310 and a patient monitoring device 320. The patient controlled input device 310 is provided with the patient 300. The patient controlled input device 310 acts an interface for the patient 300 to communicate his pain level. By activating the patient controlled input device 310, the patient 300 requests for analgesic drug. The patient monitoring device 320 is configured to monitor patient 300 for identifying the pain level of the patient. The patient monitoring device 320 may measure various physiological parameters of the patient 300 and from the measured parameter the pain level of the patient is determined. The patient monitoring device 320 is activated after administering first dosage of drug to the patient 300. Continuous monitoring of the patient 300 will help in identifying the effectiveness of the drug on the patient 300. The patient monitoring device 320 could be integrated with the existing PCA device.

The PCA device is further provided with a processor 330 configured to receive the pain level from the patient monitoring device 320 and the drug demand from the patient controlled input device 310. The patient controlled input device 310 could be connected to the processor 330 through a cable or communicates wirelessly to the device. The processor 330 could be software or hardware implemented. Dedicated hardware may be used instead of software and/or firmware for performing information processing, or a combination of dedicated hardware and software, or software in combination with a general purpose processor or a digital signal processor may be used. Once the requirements for such software and/or hardware and/or dedicated hardware are gained from an understanding of the descriptions of embodiments of the invention contained herein, the choice of any particular implementation may be left to a hardware engineer and/or software engineer. However, any dedicated and/or special purpose hardware or special purpose processor is considered subsumed in the block labeled processor 330. In an embodiment, the processor 330 automatically calculates the drug dosage upon obtaining the drug demand and the patient pain level.

The PCA device is further associated with a drug delivery device 340. The drug delivery device 340 is configured to administer drug to the patient 300 under instructions from the processor 330. The drug delivery device 340 may automatically administer the drug to the patient 300 under the instructions from the processor 330.

The PCA device further comprises a PCA interface 350 configured to display and interact with the user or the caretaker. The PCA interface 350 comprises a patient parameter interface 351. The patient parameter interface 351 could assist the PCA device in identifying which parameters need to be measured. The pain level of the patient or the effectiveness of the drug could be calculated by measuring different physiological parameters. The selection of the patient parameter that needs to be measured is done through the patient parameter interface 351 and the clinician may select these parameters from the patient parameter interface 351. Different patient parameter such as SSI, heart rate etc could be monitored. The patient parameter measured could be displayed on a display 352 associated with the PCA interface 350.

The PCA interface 350 further includes a control panel 353 for facilitating human intervention in controlling the drug administration. The processor 330 could display the calculated drug dosage on the display 352. Based on the same, a clinician or caretaker may provide his inputs or initiate the drug administration. The clinician may instruct the processor 330 through the control panel 353. A drug delivery interface 354 in the PCA interface 350 assist a clinician in selecting the dosage calculated by the processor 330 manually. The drug delivery interface 354 facilitate selection of drug dosage, time etc. It also helps in assisting the clinician in controlling the drug administration by having " start" and "stop" button to control the drug delivery.

The PCA interface 350 may also have user interface 355 for facilitating interaction of a clinician or caretaker with the PCA device. The PCA interface 350 could further comprise a threshold setting interface 356 and the clinician or caretaker could adjust various threshold levels such a patents' maximum and minimum pain threshold level, maximum or minimum drug level in blood etc. In an embodiment, processor 330 may automatically adjust certain threshold values.

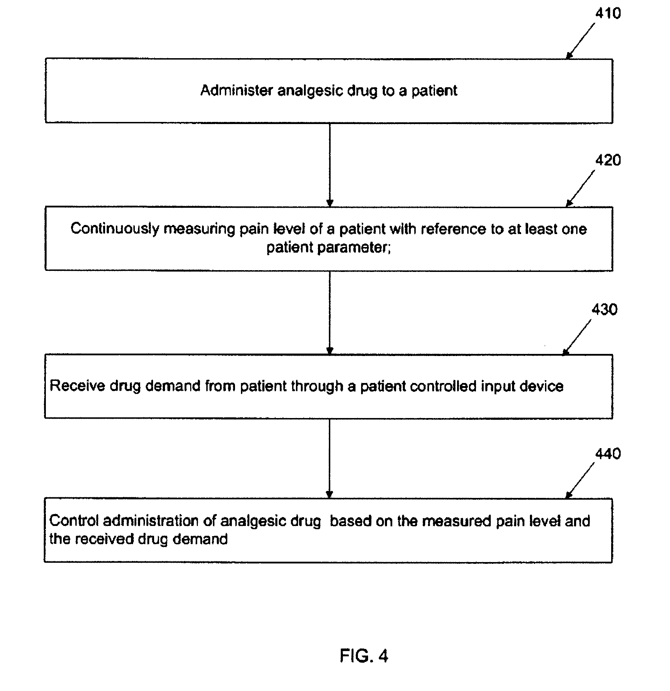

**FIG. 4** is a flowchart illustrating an analgesic drug delivery method as described in an embodiment of the invention. At step 410, analgesic drug is administrated to the patient. Initially this could be done using standard dosage depending on the clinical condition of the patient. Alternately, the patients SSI level or pain level could be considered, if available. Generally, the clinician using his diligence administers the initial dosage of drug. Alternately, the patient may be provided with a patient controlled input device configured to communicate the patient drug demand from the patient. Once the drug is administered, the patient's pain level is continuously monitored, as at step 420. The pain level is monitored by measuring certain physiological parameters of the patient. Alternately, surgical stress Index or any other pain level indicating parameter may be measured. From the measured physiological parameter pain level is detected. In an embodiment, from the measures pain level, the effectiveness of the drug could be calculated. At step 430, a drug demand is received from the patient. The patient is provided with the patient controlled input device and patient activates the same upon feeling the pain. At step 440, the analgesic drug is administered to the patient, the administration of the drug is being controlled based on the drug demand from the patient and the measured pain level.

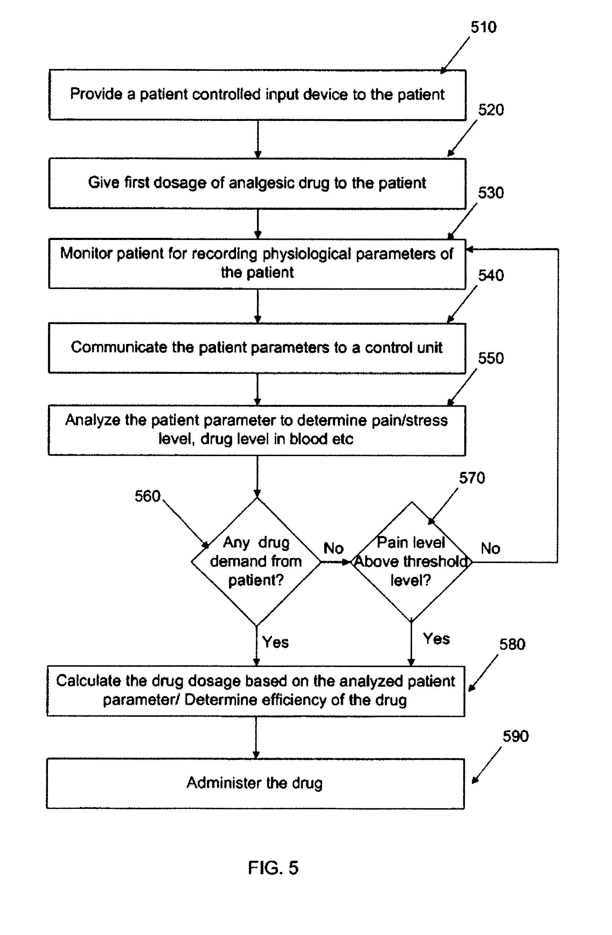

**FIG. 5** is a flowchart illustrating an analgesic drug delivery method as described in another embodiment of the invention. At step 510, a patient controlled input device is provided to the patient. This is a communication device and by activating the same, the patient can communicate a drug demand. At step 520, first dosage of analgesic drug is given to the patient. This dosage could be decided by a clinician based on the patient's medical condition or any other relevant information. At step 530, patient is monitored and his physiological parameters are recorded. The patient's pain level could be identified from the measured physiological parameters. At step 540, the measured parameters are communicated to a control unit. At step 550, the control unit analyzes the measured physiological parameter. The patient's pain level is identified from the measured physiological parameter. Further the effectiveness of the drug could also be calculated from the physiological parameters. At step 560, a check is made to identify whether any drug demand has been received from the patient. If there is a drug demand, based on the measured pain level or effectiveness of the drug, the dosage of the drug is calculated at step 580 and drug is administrated to the patient at step 590. If there is no drug demand from the patient, a comparison is made between the measured pain level and the patient's maximum pain level threshold, as at step 570. If the measured pain level is higher than the maximum threshold pain level, the control unit may administer the drug automatically to the patient. If the pain level is within the maximum pain level threshold, the system may continue with monitoring of the patient parameters, as at step 530.

In yet other embodiments of the present invention, a machine readable medium or media may include, but not limited to, magnetic disks and diskettes, optical disks and diskettes, and/or ROM, flash ROM, and/or battery backed RAM, or any other suitable magnetic, optical, or electronic medium or media. The medium (or media) has recorded thereon instructions configured to instruct a system that includes a computer, memory, and a display. The instructions include instructions for continuously measuring pain level of a patient with reference to at least one patient parameter, after administering an analgesic drug to a patient and instructions for controlling administration of analgesic drug based on the measured pain level and a drug demand received from the patient.

However software and/or firmware (hereinafter referred to generically as "software") can be used to instruct the computer to perform the inventive combination of actions described herein. Further, in some embodiments, this may comprise one or more electronic hardware components or special-purpose hardware components that may be configured to perform the same purpose as a software module or to aid in the performance of the software module.

Some of the advantages of the invention include providing faster, more stable, and accurate control of analgesic drugs, thus leading to patient well-being, decreased drug usage, and better recovery.

The above-description of the embodiments of the methods and systems has the technical effect automatically controlling the administration of analgesic drug based on the effectiveness of the drug.

As used herein, an element or step recited in the singular and proceeded with the word "a" or "an" should be understood as not excluding plural elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property. Moreover, the terms "computer" and "processor" are used interchangeably herein to refer to either specialized hardware to perform digital signal processing, control, data manipulation, and/or calculations, or a general purpose computer that can be programmed to perform the same functions and/or adapted to interface with external digital signals. The phrases "computer or processor" and "processor or computer" are therefore intended to have equal scope with either of the individual terms and are not intended to imply a dichotomy between the two terms.

Exemplary embodiments are described above in detail. The assemblies and methods are not limited to the specific embodiments described herein, but rather, components of each assembly and/or method may be utilized independently and separately from other components described herein. Further the steps involved in the workflow need not follow the sequence in which there are illustrated in figures and all the steps in the work flow need not be performed necessarily to complete the method.

While the invention has been described with reference to preferred embodiments, those skilled in the art will appreciate that certain substitutions, alterations and omissions may be made to the embodiments without departing from the spirit of the invention. Accordingly, the foregoing description is meant to be exemplary only, and should not limit the scope of the invention as set forth in the following claims.
Various aspects of the present invention are defined in the following numbered clauses:
1. A method of delivering an analgesic drug comprising:
   administering an analgesic drug to a patient based on input from a patient-controlled input device;
   continuously measuring pain level of the patient with reference to at least one patient parameter;
   receiving drug demand from the patient through the patient-controlled input device; and
   controlling administration of the analgesic drug based on the measured pain level and the received drug demand.
2. A method as claimed in clause 1, wherein the method includes: controlling the drug delivery automatically upon the measured pain level reaching a maximum threshold value of pain level.
3. A method as claimed in clause 1 or clause 2, wherein the method includes:
   controlling the drug administration using the measured pain level and preceding drug demands.
4. A method as claimed in clause 2, wherein the method includes: controlling the pain threshold level using the measured pain level and preceding drug demands.
5. A method as claimed in any preceding clause, wherein the step of measuring pain level includes: deriving pain level from at least one of the measured Surgical Stress Index (SSI), heart rate, skin conductivity, blood pressure, pupil size and forehead muscle activity.
6. A method as in any preceding clause, wherein the method further comprises:
   monitoring the gestures of a patient for determining pain level and for identifying drug demand from the patient.
7. A method as claimed in clause 6, wherein the step of monitoring gestures comprises: monitoring at least one of facial expressions, sound, movements of body parts, activity, cry, and consolability of the patient.
8. A method as claimed in any preceding clause, further comprising: obtaining a minimum threshold value of a patient parameter and controlling the drug administration based on the current value of the patient parameter.
9. A method as claimed in any preceding clause, wherein the method includes:
   configuring personal threshold of a patient parameter with reference to at least one of monitored patient parameter including cardiac rhythm, trends of SSI and drug concentration.
10. An analgesic drug delivery system comprising:
   a drug demand interface obtaining a drug demand request from a patient;
   a patient parameter monitoring device measuring pain level of the patient;
   a control system configured to receive input from the patient parameter monitoring device and the drug demand interface and configured to control the drug delivery based on effectiveness of the drug; and
   a drug delivery system configured to administer the drug in a controlled manner under the control of the control system.
11. The system as claimed in clause 10, wherein the drug demand interface is part of a patient controlled analgesia (PCA) device configured to communicate the drug demand request from the patient.
12. The system as claimed in clause 10 or clause 11, wherein the patient parameter monitoring device includes pain level monitoring devices using SSI, heart rate, patient gestures, skin conductivity, blood pressure, pupil size and forehead muscle activity.
13. The system as claimed in any of clauses 10 to 12, wherein the control system is configured to calculate the effectiveness of the drug from the measured pain level.
14. The system as claimed in clause 13, wherein the control system calculates the effectiveness of the drug by continuously monitoring the pain level after administering the drug and by analyzing the previous drug demand requests and the current drug level in blood.
15. An improved patient controlled analgesia (PCA) device comprising:
   a patient controlled input device associated with a patient configured to communicate a patient request about drug demand;
   a patient monitoring device for measuring pain level of the patient continuously;
   a processor for checking effectiveness of the drug and processing the patient request with reference to the measured pain level and effectiveness of the drug;
   a drug delivery device associated with the processor configured to administer the drug under the control of the processor such that the drug is administered to the patient before the patient is uncomfortable.
16. The device as claimed in clause 15, wherein the patient monitoring device includes: monitoring the pain level and converting the pain level into a numerical parameter.
17. The device as claimed in clause 15 or clause 16, wherein the patient request device is configured to communicate the drug requirement to the patient.
18. The device as claimed in any of clauses 15 to 17, wherein the processor is configured to calculate the drug dosage based on the measured pain level and the previous drug demands.
19. The device as claimed in any of clauses 15 to 18, wherein the processor is configured to control the drug dosage based on personal threshold.
20. The device as claimed in any of clauses 15 to 19, wherein the device further comprises a PCA interface.

## Claims

1. An analgesic drug delivery system comprising:
a drug demand interface (110) obtaining a drug demand request from a patient;
a patient parameter monitoring device (120) measuring pain level of the patient;
a control system (130) configured to receive input from the patient parameter monitoring device (120) and the drug demand interface (110) and configured to control the drug delivery based on effectiveness of the drug; and
a drug delivery system (140) configured to administer the drug in a controlled manner under the control of the control system (130).

2. The system as claimed in claim 1, wherein the drug demand interface (110) is part of a patient controlled analgesia (PCA) device configured to communicate the drug demand request from the patient.

3. The system as claimed in claim 1 or claim 2, wherein the patient parameter monitoring device (120) includes pain level monitoring devices using Surgical Stress Index (SSI), heart rate, skin conductivity, blood pressure, pupil size and forehead muscle activity.

4. The system as claimed in any preceding claim, wherein the control system (130) is configured to calculate the effectiveness of the drug from the measured pain level.

5. The system as claimed in claim 4, wherein the control system (130) calculates the effectiveness of the drug by continuously monitoring the pain level after administering the drug and by analyzing the previous drug demand requests and the current drug level in blood.

6. The system as claimed in any preceding claim, wherein the patient parameter monitoring device (120) monitors gestures of the patient.

7. The system as claimed in claim 6, wherein the patient parameter monitoring device (120) also identifies drug demand from the patient.

8. The system as claimed in claim 6 or claim 7, wherein the patient parameter monitoring device (120) monitors gestures of the patient by monitoring at least one of facial expressions, sound, movements of body parts, activity, cry and consolability of the patient.

9. The system as claimed in any preceding claim, wherein the control system (130) controls the drug delivery based on a minimum threshold value of a patient parameter.

10. The system as claimed in any preceding claim, wherein the pain level is measured continuously.

11. A method of delivering an analgesic drug comprising:
administering an analgesic drug to a patient based on input from a patient-controlled input device;
continuously measuring pain level of the patient with reference to at least one patient parameter;
receiving drug demand from the patient through the patient-controlled input device; and
controlling administration of the analgesic drug based on the measured pain level and the received drug demand.

12. A method as claimed in claim 11, wherein the method includes: controlling the drug delivery automatically upon the measured pain level reaching a maximum threshold value of pain level.

13. A method as claimed in claim 11 or claim 12, wherein the method includes:
controlling the drug administration using the measured pain level and preceding drug demands.

14. A method as claimed in claim 12, wherein the method includes: controlling the pain threshold level using the measured pain level and preceding drug demands.

15. A method as claimed in any of claims 11 to 14, wherein the step of measuring pain level includes: deriving pain level from at least one of the measured Surgical Stress Index (SSI), heart rate, skin conductivity, blood pressure, pupil size and forehead muscle activity.
